# FASCICULE DE BREVET EUROPEEN

(11) **EP 2 091 663 B1**
(45) Date de publication et mention de la délivrance du brevet: **05.09.2012**
(21) Numéro de dépôt: 07822511.7
(22) Date de dépôt: 12.11.2007
(51) Int. Cl.: B05B 17/06

(54) **ATOMISEUR DE LIQUIDE PAR ULTRASONS**
ULTRASCHALLFLÜSSIGKEITSZERSTÄUBER
ULTRASOUND LIQUID ATOMISER

(30) Priorité: 14.11.2006 FR 0609905
(43) Date de publication de la demande: 26.08.2009
(73) Titulaire: Telemaq, 06370 Mouans-Sartoux (FR)
(72) Inventeur: SAUZADE, Jean-Denis, 06130 Grasse (FR)
(74) Mandataire: Grosfillier, Philippe
(86) Numéro de dépôt international: PCT/EP2007/062234
(87) Numéro de publication internationale: WO 2008/058941

(56) Documents cités:
- EP-A- 1 386 672
- EP-A1- 0 432 992
- WO-A-93/10910
- US-A- 4 585 167

## Description

### Domaine de l'invention

La présente invention concerne les distributeurs de fluide, communément nommés atomiseurs, qui permettent de diffuser de manière précise, au moyen d'éléments piézoélectriques, des fluides sous forme de micro gouttelettes ou d'aérosols.

### Etat de la technique

La distribution de fluides ou de liquides, quelle que soit leur nature (huileuse, aqueuse ou alcoolique), qu'il s'agisse de solutions ou de suspensions (particules en suspension dans un liquide), s'effectue dans un grand nombre d'applications par micronisation, atomisation, nébulisation ou génération d'aérosols. Les principales applications utilisant ces dispositifs de distribution de fluide concernent l'administration de médicaments (pharmacie), la diffusion de produits cosmétiques (en particulier les parfums), la désinfection, la génération d'odeur, l'humidification de l'air ou de supports (papier, textiles...) et la distribution de réactifs biologiques.

En ce qui concerne les applications médicales, des nébuliseurs sont employés depuis des décennies pour délivrer des médicaments par inhalation. Les dispositifs utilisés à cet effet peuvent comporter une pompe doseuse mécanique, un nébuliseur pneumatique ou ultrasonore. Jusqu'à récemment, ces dispositifs étaient destinés à délivrer des médicaments par les voies respiratoires, à un niveau relativement superficiel. Depuis quelques années, l'industrie pharmaceutique s'intéresse à l'administration de médicaments sous forme d'aérosols au plus profond des poumons afin d'atteindre les bronchioles. En agissant de la sorte, il deviendrait possible d'administrer par voie respiratoire des médicaments systémiques ou des gènes.

Pour ce faire, il est nécessaire de développer de nouvelles technologies pour améliorer le rendement, la précision et l'homogénéité des aérosols destinés à se déposer dans les bronchioles. Les dispositifs traditionnels sont, de manière générale, limités soit par une trop grande vitesse d'expulsion de l'aérosol, soit par une trop forte consommation, soit par une dégradation des produits médicamenteux, soit par une trop grande taille de gouttelettes, soit par le bruit.

Pour satisfaire les nouvelles conditions exigées par la délivrance de médicaments par voie respiratoire, plusieurs fabricants de générateurs d'aérosols ont développé des dispositifs basés sur des grilles ou des membranes micro perforées vibrantes. Parmi ces fabricants, on peut citer Nektar (Aeroneb), Odem (TouchSpray), Pari (e-Flow), Pfeiffer (MicroHaler), Omron (NE-UO22), Sheffield Pharmaceutical (Premaire), Alexza (Staccato).

Les premières études relatives à des atomiseurs ultrasonores comportant une membrane micro perforée vibrante ont été réalisées par le laboratoire de recherche de Matsushita dans les années 1980. Le principe de l'éjection de liquide en fines gouttelettes au travers d'une membrane vibrante micro perforée soumise à vibration est particulièrement développé dans les publications suivantes : Ueha S., et al. Mechanism of ultrasonic atomisation using a multi-pinhole plate. Acoust.Soc. Jpn. (E) 6,1:21 (1985). Maehara N., et al. Influence of the vibrating system of a multipinhole-plate ultrasonic nebulizer on its performance. Review of scientific instruments, 57 (11), Nov. 1986, pp.2870-2876. Maehara N., et al. A pinhole-plate ultrasonic atomizer. Ultrasonics Nov. 1984.

L'ensemble de ces études à fait l'objet de plusieurs brevets, notamment US 4 533 082 (1985) et US 4 605 167 (1986) qui décrivent un atomiseur utilisant une membrane vibrante perforée de micro trous. La membrane comporte une partie courbe (protubérance ou dôme) en son centre permettant de faire diverger les gouttelettes générées. La membrane comporte des micro-perforations de 30 à 100 µm de diamètre. L'élément vibrant est une céramique piézoélectrique annulaire de diamètre extérieur de 5 à 15 mm, de diamètre intérieur de 2 à 8 mm. La membrane vibrante d'une épaisseur de 30 à 120 µm est collée sur la céramique annulaire. Cette céramique piézoélectrique est excitée à des fréquences comprises entre 30 et 200 kHz sur son mode de déformation radiale. Le brevet Bespack US 5 152 456 (1992), voir également la demande européenne correspondante, EP 0 432 992 A1, en accord avec le préambule de la revendication 1, reprend le principe proposé par Matsushita. Cependant, l'élément vibrant (ou vibrateur) est constitué d'un disque annulaire en aluminium de 22 mm de diamètre. La céramique piézoélectrique est fixée sur ce disque en aluminium. Le mode de fonctionnement de la céramique piézoélectrique correspond à une déformée radiale. L'ouverture centrale du disque en aluminium est de 4 mm. La membrane en Nickel comporte 1500 perforations (ou trous) de 3 µm de diamètre.

Le brevet Toda US 5 297 734 (1994) fait référence à un atomiseur de type lame vibrante de géométrie carrée permettant des débits jusqu'à 1 l/heure. L'atomiseur est constitué d'une membrane en Nickel de 50 µm d'épaisseur collée sur un disque de céramique piézoélectrique de diamètre extérieur de 24 mm, de diamètre intérieur de 12 mm et d'épaisseur 6 mm. La membrane dispose de trous coniques de diamètre intérieur 1 mm et de diamètre extérieur de 20 µm.

Le brevet de Technology Transfer Partnership (TTP) US 5 261 601 (1993) décrit un atomiseur basé sur le brevet Bespack précité. Le brevet TTP US 5 518 179 (1996) fait quant à lui référence à un atomiseur disque ou la membrane perforée est en Nickel électro-formée. L'atomiseur TTP ne requiert pas de chambre de liquide derrière la membrane et l'alimentation en liquide s'effectue par capillarité (utilisation de mèche ou de matière poreuse). L'atomiseur TTP met en avant sa structure bimorphe précisant le mode de flexion de l'ensemble composé de la céramique piézoélectrique et de la membrane micro perforée. La membrane est de rigidité comparable à celle de la céramique piézoélectrique annulaire. L'atomiseur de référence est constitué d'un anneau de laiton de diamètre extérieur de 20 mm et d'épaisseur 200 µm. L'anneau de céramique piézoélectrique a un diamètre extérieur de 20 mm, un diamètre intérieur de 6 mm et une épaisseur de 200 µm. Le brevet US 6 085 740 (2000) d'Aerogen mentionne un moyen pour atomiser un liquide en fines gouttelettes en utilisant une micro-grille. La membrane, munie de micro-trous de 1 à 6 µm de diamètre, est mise en vibration par un bilame piézoélectrique fonctionnant à 45 kHz. Le liquide est alimenté par capillarité et la membrane est dissociable du vibrateur.

Le brevet US 6 427 682 (2002) d'Aerogen décrit la réalisation d'un appareil de diffusion de médicaments utilisant une membrane vibrante. Son principe est très proche de celui de Matsushita. Il est constitué d'une pièce en aluminium mise en vibration par flexion à l'aide d'une céramique piézoélectrique annulaire. La membrane vibrante munie de micro-trous est réalisée par électroformage. Une chambre, contenant le liquide est en contact avec la membrane.

Par ailleurs, Omron a développé une technologie de pompe ultrasonore permettant d'atomiser un liquide au travers d'une membrane micro perforée. Cette technologie est décrite dans le brevet US 6 901 926 (2005). Dans la technologie Omron, la membrane micro perforée n'est pas directement mise en vibration par l'élément vibrant. Les gouttelettes sont formées par éjection du liquide à travers les trous lors de la variation de pression dynamique due à la pompe ultrasonore.

Les atomiseurs piézoélectriques ou ultrasonores de l'état de la technique (fréquence supérieure à 20 kHz) qui comprennent une membrane micro perforée soumise à vibration fonctionnent tous par flexion de la membrane et de la céramique piézoélectrique qui lui est associée. Il s'agit en fait de combiner de différentes manières une céramique piézoélectrique annulaire et une membrane métallique de faible épaisseur comportant un grand nombre de micro perforations. Ce type de structure bénéficie d'une faible épaisseur.

Les dispositifs de l'état de la technique possèdent cependant plusieurs inconvénients.

Un premier inconvénient réside dans le fait que la membrane micro perforée participe fortement au mode de résonance de l'atomiseur. Ce phénomène a pour effet que l'atomiseur est lui même fortement amorti dans sa résonance par le liquide qui est en contact avec la membrane. Le fonctionnement de l'atomiseur dépend fortement de la quantité de liquide ou de la pression exercée par le liquide derrière la membrane. Ceci se traduit par une complication dans le pilotage de la fréquence d'excitation de l'atomiseur. De plus, cet amortissement conduit à un échauffement de l'élément vibrant et de la membrane. Cet échauffement a pour conséquence de limiter le temps de fonctionnement de l'atomiseur, d'augmenter la consommation électrique nécessaire au fonctionnement de celui-ci et peut conduire à une dégradation des propriétés du liquide à atomiser. Par ailleurs, dans ce type de structure, la déformation de la membrane en flexion ne permet pas d'obtenir un déplacement homogène sur l'ensemble de la surface de la membrane. Suivant l'emplacement des micro-trous sur la membrane, ceux-ci n'ont pas le même débit et la génération d'aérosol peut s'avérer instable (effet de seuil). D'autre part, la sensibilité de la structure vibrante aux fixations mécaniques et aux étanchéités au liquide (amortissements parasites) rendent complexes et coûteuses les solutions technologiques pour mettre en oeuvre industriellement de telles structures en grande quantité et à faible coût.

Il existe donc un besoin de remédier à ces différents inconvénients et de proposer des dispositifs plus robustes, plus faciles à piloter électroniquement, plus économes en énergie, plus faciles à industrialiser en grande quantité et de faible coût.

### Description générale de l'invention

La présente invention se propose de remédier notamment aux problèmes exposés dans le chapitre précédent.

A cet effet, elle a pour objet un atomiseur tel que définit dans la revendication principale.

Des modes de réalisation préférentiels font l'objet des revendications dépendantes.

Dans le présent texte, par « transducteur », on entend un élément constitué d'un corps de transducteur piézoélectrique, d'au moins un élément piézoélectrique et optionnellement d'une masse arrière.

Par « section », on entend une figure géométrique constituée par l'intersection d'un plan et d'un volume. Ainsi, en considérant par exemple un objet cylindrique dont l'intérieur a un diamètre variable, on dira qu'il possède une section variable sur sa longueur.

Le corps de transducteur comporte un axe de symétrie.

Plusieurs avantages offerts par l'atomiseur selon la présente invention résultent du fait que le corps de transducteur piézoélectrique sur lequel est fixée la membrane vibre selon un mode longitudinal, c'est-à-dire, selon une direction parallèle à l'axe de symétrie du corps du transducteur piézoélectrique.

Un ou plusieurs éléments piézoélectriques peuvent être prévus.

De préférence, la section du corps de transducteur piézoélectrique varie sur sa longueur.

Selon un mode de réalisation, la section varie de manière discontinue.

Avantageusement, la section varie brusquement en un seul endroit.

Une telle variation de section est illustrée dans le mode de réalisation suivant dans lequel le corps de transducteur piézoélectrique comporte deux parties dont le diamètre externe diffère. La zone d'amplification de déformations dont l'extrémité comprend la membrane, possède le diamètre le plus faible. Dans cette configuration, nommée « trompe » dans le présent texte, les ondes ultrasonores longitudinales sont amplifiées en déplacement lors du changement de section du transducteur. L'élément tubulaire joue le rôle d'amplificateur de déplacements longitudinaux microniques.

Selon un autre mode de réalisation de l'invention, la membrane forme au moins partiellement un dôme qui réalise plusieurs fonctions. De manière commune à l'état de l'art antérieur, la membrane micro-perforée est conçue pour retenir le liquide dans la chambre d'atomisation à l'arrière de la membrane et de contenir la pression statique de celui-ci. L'équilibre des pressions, la forme des trous et la nature du matériau utilisé pour la membrane est ainsi fait que le liquide ne suinte pas à l'extérieur de la membrane et qu'aucun phénomène de « gouttage » ou de perte de liquide n'apparaît. Par ailleurs, la forme de dôme permet de mieux répartir le brouillard de micro-gouttelettes ou d'aérosol en faisant diverger le jet par simple effet géométrique. D'autre part, la vitesse vibratoire associée au déplacement de la membrane, permet d'éjecter les micro-gouttelettes à travers les trous. Dans le cas de la présente invention, les dimensions du dôme et de la membrane micro-perforée sont telles que le dôme joue un effet d'amplification de mouvement vibratoire tout en conservant une répartition de vitesse vibratoire homogène sur la surface de la membrane. Contrairement à l'état de l'art antérieur, la membrane n'influence pas le comportement vibratoire du transducteur. Quelle que soit l'épaisseur de la membrane (par exemple de 20 à 200 µm), l'élément piézoélectrique conserve ses caractéristiques dynamiques et ses performances vibratoires. Plus spécifiquement, la fréquence de résonance et les déplacements vibratoires du transducteur ne sont pas modifiés par le couplage mécanique de la membrane. Ceci procure des avantages à l'atomiseur dans la mesure où la conception du transducteur (fréquences, déplacements vibratoires, modes de déformation, coefficient de couplage et facteur de qualité mécanique) peut être effectuée sans tenir compte de la membrane (géométrie et matière).

Cette propriété de la présente invention permet avantageusement d'optimiser la structure du transducteur (ou convertisseur) afin de favoriser soit la vitesse de sortie de l'aérosol, soit le débit, soit la fréquence de résonance, soit la consommation ou le rendement du transducteur. De cette manière, il est possible de réaliser des atomiseurs à membrane vibrante permettant la production d'aérosols à vitesse quasi nulle (application médicale) jusqu'à des vitesses d'éjection par exemple de l'ordre de 30 m/s (application cosmétique). De même, le débit d'atomisation n'est plus lié directement à la surface de la céramique piézoélectrique mais à la longueur du transducteur piézoélectrique permettant d'adapter des débits de 1 µl/s à 300 µl/s. C'est cette même longueur qui gouverne directement la fréquence de résonance de l'atomiseur. Le mode de fonctionnement de l'atomiseur n'est pas un mode de flexion mais un mode d'élongation longitudinal. Ceci permet de travailler à de hautes fréquences (50 à 200 kHz) avec des céramiques piézoélectriques de faible diamètre sans pénaliser le débit de liquide et surtout avec de très faibles pertes. Ces pertes mécaniques qui correspondent à une énergie dissipée en chaleur dans le transducteur s'élèvent fortement en fonction de la fréquence de fonctionnement du transducteur. Dans la présente invention, celles-ci sont réduites car les rendements des structures de type transducteur longitudinal sont bien meilleurs que les structures de type « bilame » fonctionnant en flexion.

Les faibles pertes dans le transducteur conduisent à la conception d'atomiseurs dont la consommation électrique est faible. Cet avantage est considérable dans la mesure où les atomiseurs de l'état de l'art antérieur sont limités dans leur application par la durée d'atomisation et la durée de vie des piles ou des batteries d'alimentation. En effet, les applications avantageuses des atomiseurs à membrane micro-perforée vibrante concernent essentiellement des dispositifs « à main » fonctionnant sur piles ou batteries. La structure, objet de la présente invention, est moins amortie par le liquide en arrière de la membrane. Cette propriété permet un échauffement moindre du liquide à atomiser. Dans les applications ou les atomiseurs délivrent des formulations médicamenteuses, un échauffement du liquide peut être rédhibitoire et limiter très fortement l'intérêt de tels atomiseurs.

Par ailleurs, selon un mode de réalisation préféré, la présente invention se caractérise par le fait que les noeuds de vibration sont accessibles pour permettre aisément la fixation mécanique de l'atomiseur. Les atomiseurs de l'état de la technique fonctionnant par flexion de la céramique annulaire sont difficiles à fixer mécaniquement sans perturber et amortir fortement le mode de vibration. Dans le cas de la présente invention, l'atomiseur est de préférence fixé mécaniquement ou surmoulé dans la zone du noeud de vibration (ce noeud est unique) et permet des solutions de montage mécanique et d'étanchéité très simples et très bon marché.

Une autre caractéristique de l'invention est que le liquide peut être directement mis en contact avec la face arrière de la membrane. En effet, le mode de vibration de l'atomiseur suivant un mode longitudinal est peu sensible à la présence d'un liquide et au poids de la colonne de ce liquide.

Cette propriété permet à l'atomiseur de fonctionner indifféremment suivant un angle qui peut varier de la verticale à l'horizontale.

Le liquide peut aussi être guidé jusqu'à la membrane par capillarité en utilisant des canaux, des mèches ou des matériaux poreux adéquats. De cette manière, le réservoir de liquide peut se situer au dessus, autour ou en dessous de l'atomiseur.

Selon une variante de l'invention, la structure de l'atomiseur comprend une masse arrière, nommée également masse dynamique, dont le rôle est d'empêcher le transducteur de vibrer à l'arrière, deux céramiques piézoélectriques reliées par une connectique commune, un amplificateur de mouvement vibratoire comportant une cavité et une membrane micro-perforée de forme variée mais préférentiellement bombée formant une protubérance ou dôme.

Selon une autre variante de l'invention, un mécanisme de précontrainte comme une vis permet de lier mécaniquement la masse arrière, les céramiques et l'amplificateur. L'amplificateur est métallique et préférentiellement en acier inox, en titane ou en aluminium. La membrane micro-perforée est collée sur l'amplificateur vibratoire. La membrane, qui influe peu sur le comportement vibratoire de l'atomiseur, peut être réalisée en matériaux variés comme du plastique, du silicium, de la céramique mais est préférentiellement réalisée en métal. Les micro-perforations peuvent être réalisées par différents moyens mais préférentiellement par électroformage ou par laser. La quantité de micro-perforations peut aller de un trou pour la distribution de liquide à la demande, à plusieurs milliers de trous. La taille des micro-trous ou la maille des grilles peuvent aller de 1 µm jusqu'à 100 µm en diamètre équivalent suivant les applications de l'atomiseur. Il a été démontré que les performances en débit et homogénéité du jet de gouttelettes de l'atomiseur étaient fortement dépendantes du mode de déplacement de la membrane. Ces performances sont améliorées si la membrane se déplace en mode « piston » sans déformation en flexion. Dans l'état de la technique, les atomiseurs fonctionnent en flexion et conduisent donc à un ou plusieurs noeuds de vibration à l'intérieur de la membrane. Dans la présente invention, la membrane ne participe pas au mode de vibration du transducteur et il est possible de dimensionner et d'optimiser la géométrie de la membrane afin que celle-ci se déforme en mode « piston ». Pour ce faire, les modélisations et les résultats d'essais montrent que le diamètre de la protubérance (ou dôme) doit être proche du diamètre de la cavité contenant le liquide (ou chambre). Ceci implique que la hauteur maximale du dôme doit être proche de la moitié du diamètre intérieur de la cavité.

Le déplacement de la membrane en mode « piston » permet d'homogénéiser la vitesse vibratoire sur la surface de la membrane. Il en résulte que les micro-perforations (ou trous) éjectent des micro-gouttelettes de tailles mieux calibrées et avec un débit identique lorsqu'on les compare entre elles.

D'autres modes de réalisation de l'invention sont brièvement exposés ci-dessous :
- Le ou les éléments piézoélectriques sont représentés par une seule céramique fixée avec une colle de rigidité suffisante sur le transducteur soumis à mouvement vibratoire.
- La céramique piézoélectrique est un multicouche permettant une alimentation électrique de faible tension (1 à 15 V_{DC}) comme peuvent en fournir, par exemple les sociétés Epcos, Fuji, Noliac, Morgan Matroc ou Physic Instruments.
- La céramique piézoélectrique est fixée sur le corps du transducteur de telle manière qu'il n'y ait aucun contact entre celle-ci et le liquide à atomiser. Cette configuration permet d'isoler électriquement la céramique piézoélectrique et élimine tout problème d'étanchéité et de compatibilité (application médicale) avec le liquide.
- L'amplificateur de déplacement vibratoire (ou « trompe ») de l'atomiseur comporte des passages, rainures ou trous de telle manière que la chambre ou le réservoir de liquide est situé autour de la trompe.
- Le transducteur piézoélectrique de l'atomiseur comporte un « pavillon » à l'extrémité de la «trompe» qui vibre préférentiellement suivant un mode « piston » sans aucune flexion. Ce « pavillon » a l'avantage d'amplifier le déplacement vibratoire du transducteur mais aussi de pouvoir fixer une membrane micro-perforée de plus grand diamètre. Cette configuration est destinée à augmenter le débit de l'atomiseur malgré sa petite dimension.
- Le transducteur piézoélectrique, fonctionnant en mode longitudinal, fait vibrer et se déformer une membrane micro-perforée ou une grille de forme cylindrique. Pour optimiser le déplacement de la membrane cylindrique qui est placée entre le transducteur et le pavillon, une protubérance peut être ajoutée afin de rigidifier la membrane de manière adéquate.
- L'atomiseur comporte une « trompe » de forme particulière comme, par exemple, un tronc de cône permettant une amplification de mouvement vibratoire par changement brutal de section. Dans cette configuration particulière, le ratio de la distance membrane-élément piézoélectrique sur le diamètre de l'élément piézoélectrique est de préférence supérieur à 0,5.
- La membrane micro-perforée ou grille n'est pas collée mais couplée acoustiquement au transducteur par des moyens mécanique de pression.
- La fixation de l'atomiseur sur un support extérieur est réalisée par un clinquant ou un circuit souple fixé par des moyens mécaniques ou par collage sur l'électrode de la céramique qui n'est pas associée au transducteur. Ce mode de fixation particulier est intéressant par sa simplicité de mise en oeuvre et son faible coût. Cette configuration a l'avantage de découpler le transducteur du support extérieur (tenue statique) et de ne pas perturber son fonctionnement dynamique. Par ailleurs, le clinquant métallique (ou le circuit souple) permet d'alimenter électriquement la céramique piézoélectrique.
- L'atomiseur est constitué d'un corps de transducteur qui comprend un moyen de fixation d'un réservoir sans perturber son fonctionnement dynamique et sans altérer ses performances.
- L'atomiseur comprend un réservoir qui est fixé mécaniquement sur le corps du transducteur sans perturber le fonctionnement de celui-ci.
- L'atomiseur comprend un organe mécanique plein ou creux réalisé en différentes matières mais préférentiellement plastique, placé coaxialement à l'intérieur de la cavité contenant le liquide, de forme varié mais préférentiellement cylindrique dont le rôle est de guider les bulles d'air qui pourraient se former au niveau de la membrane vibrante et qui bloqueraient le processus d'atomisation.
- L'atomiseur comprend un capteur de présence de liquide constitué d'une électrode placée à l'intérieur de la « trompe » et proche de la membrane vibrante. On injecte sur cette électrode un courant électrique alternatif basse fréquence dont le signal est récupéré sur la masse électrique du transducteur par traitement. Le courant alternatif se propage de l'électrode à la membrane vibrante via la conductivité du liquide. La présence ou l'absence de ce signal indique la présence ou l'absence de liquide.
- L'atomiseur est placé dans un boîtier de forme varié qui peut se tenir dans la main ou appliqué sur une partie du corps (humain ou animal) comme un masque et réalisé en différentes matières mais préférentiellement en plastique. Ce boîtier peut constituer un diffuseur de parfum, d'humidité, de désinfectant ou de médicament. Tout particulièrement, l'atomiseur associé au boîtier peut être utilisé comme un dispositif de délivrance de médicament par voie pulmonaire, nasale ou oculaire.
- L'atomiseur pourra être associé à un boîtier dédié spécifiquement à la délivrance de médicament par voie pulmonaire. Ce boîtier pourra comporter l'ensemble des fonctions permettant de gérer l'inhalation ou la diffusion de ce médicament. En particulier, il comportera un embout ou un adaptateur anatomique qui peut être jetable, un ensemble de valves ou de chicanes permettant de gérer au mieux le flux d'air (aspiré ou refoulé), un dispositif de déclenchement de l'atomisation à l'inhalation qui peut être réalisé soit mécaniquement soit électroniquement, un réservoir soit à la pression ambiante soit à atmosphère contrôlée (stérile) en dépression, un capteur de niveau de liquide et un mécanisme évitant la formation de bulles d'air tel que décrit dans ce brevet. Dans le présent texte, cet ensemble est désigné comme « inhalateur ».
- L'atomiseur, intégré dans un boîtier mécanique, peut être commandé électroniquement par un boîtier électronique extérieur permettant de l'alimenter par pile, batteries ou secteur. Ce même boîtier électronique peut être intégré dans le boîtier mécanique afin d'assurer une autonomie complète du dispositif. Ce boîtier électronique intégré pourra être alimenté par une batterie, une pile ou un super-condensateur rechargeable soit par le secteur soit par effet inductif.
- L'atomiseur comprend une fonction électronique de détartrage ou plus généralement de débouchage réalisé par un mode d'alimentation électrique particulier en appliquant des cycles de tensions électriques sur l'éléments piézoélectrique de durée, d'amplitude ou de fréquence différents de l'alimentation électronique nominale. Ce mode de débouchage pourra être réalisé alors que l'atomiseur est plongé dans un bain de produit détartrant, nettoyant ou stérilisant.
- L'atomiseur comprend une membrane comportant des trous de taille micronique dont le diamètre a été réduit par le biais d'un traitement de surface (polymère ou métallique) tout particulièrement par dépôt d'or électrolytique. Par ailleurs, ces différents traitements de surface jouent un rôle pour réduire les phénomènes de gouttage ou de bouchage et assurent, dans certains cas, des fonctions bactéricides, virucides ou de biocompatibilité.
- L'atomiseur est constitué de matériaux ou comportant un traitement de surface permettant d'assurer sa stérilisation à froid ou à chaud (étuvage). En particulier, l'atomiseur comprendra des céramiques piézoélectriques haute température (>150°C), un transducteur en acier inoxydable ou en titane, une membrane recouverte d'un flash d'or électrolytique.
- L'atomiseur est connecté électriquement de telle manière que le potentiel électrique de la membrane soit différent de la masse électrique du boîtier électronique. Cette configuration électrique permet de charger électriquement les micro-gouttelettes afin de faciliter le guidage de l'aérosol à travers le circuit aéraulique du dispositif et des voies respiratoires.
- L'atomiseur comprend un mécanisme de perforation réalisé par une canule ou aiguille creuse réalisée, par exemple, en plastique ou en métal placé au centre de l'atomiseur. Ce mécanisme peut comprendre les fonctions d'évitement des bulles d'air et de mesure de niveau de liquide. Ce dispositif de perforation permet de recevoir un réservoir ou un flacon stérile et étanche possédant un opercule de matière élastomère capable d'être perforé.
- L'atomiseur est muni d'un clinquant métallique (ou circuit souple) servant d'électrode et permettant l'alimentation de la céramique qui peut être utilisé pour réaliser un capteur de dépression associé avec la céramique piézoélectrique. La tension générée sur la céramique lors de l'inspiration peut être exploitée pour réaliser un dispositif de déclenchement de l'atomisation à l'inhalation.

Il va de soi que l'invention ne se limite pas aux modes de réalisation exposés ci-dessus. Ceux-ci ne constituent que des exemples parmi d'autres.

On notera également qu'outre l'instauration d'un mode de vibration longitudinal, il est possible de prévoir un mode de vibration radial.

### Description détaillée de l'invention

L'invention sera mieux comprise dans le présent chapitre au moyen d'une description détaillée et d'exemples non-limitatifs illustrés par des figures.

### Brève description des figures :

La figure 1 représente en coupe un exemple d'atomiseur suivant l'invention.
Les figures 2, 3, 4, 5, 6, 7, 8, 9, 10 et 13 représentent en coupe, des variantes de cet atomiseur.
Les figures 11 représentent les déformées des membranes suivant la structure des atomiseurs.
Les figures 12 représentent des modélisations du comportement vibratoire de l'atomiseur suivant la présente invention.
La figure 13 représente une coupe d'un atomiseur comprenant une membrane vibrante tubulaire placée autour de la « trompe », elle même vibrant suivant un mode longitudinal.
Les figures 14 & 15 montrent respectivement les vues en perspective et les coupes des atomiseurs tubulaires cylindriques ou en tronc de cône.
La figure 16 représente la vue en perspective et la coupe d'un inhalateur médical simple en forme de T intégrant un atomiseur tel que décrit par la présente invention.
Les figures 17A & 17B illustrent deux configurations d'un inhalateur en format « poche » intégrant un atomiseur tel que décrit par la présente invention. La figure 17A représente un inhalateur piloté par un boîtier électronique extérieur. La figure 17B représente un inhalateur dont l'électronique est intégrée dans le boîtier. La figure 17C montre une coupe de ce même inhalateur et permet de visualiser la position de l'atomiseur dans son boîtier.
La figure 18 représente la coupe d'un atomiseur comportant les différentes fonctions d'évacuation de bulles d'air et de capteur de présence de liquide.

### Liste des références numériques utilisées dans les figures :

- 1.: Corps de transducteur piézoélectrique
- 1 a.: Zone de concentration de contraintes
- 1b.: Zone d'amplification de déformations
- 2.: Céramique piézoélectrique monobloc.
- 3.: Membrane micro-perforée
- 4.: Cavité contenant le liquide
- 5.: Masse arrière
- 6.: Vis de précontrainte.
- 7.: Electrode.
- 8.: Elément de liaison.
- 9.: Céramique piézoélectrique multicouche.
- 10.: Pavillon
- 11.: Retour de masse électrique.
- 12.: Cache transducteur.
- 13.: Réservoir.
- 14.: Bouchon.
- 15.: Boîtier.
- 16.: Embout.
- 17.: Ouvertures ou valves.
- 18.: Câbles d'alimentation
- 19.: Boîtier électronique.
- 20.: Connecteur électrique.
- 21.: Tube coaxial.
- 22.: Capteur de présence de liquide.
- 23.: Câble de retour du capteur.

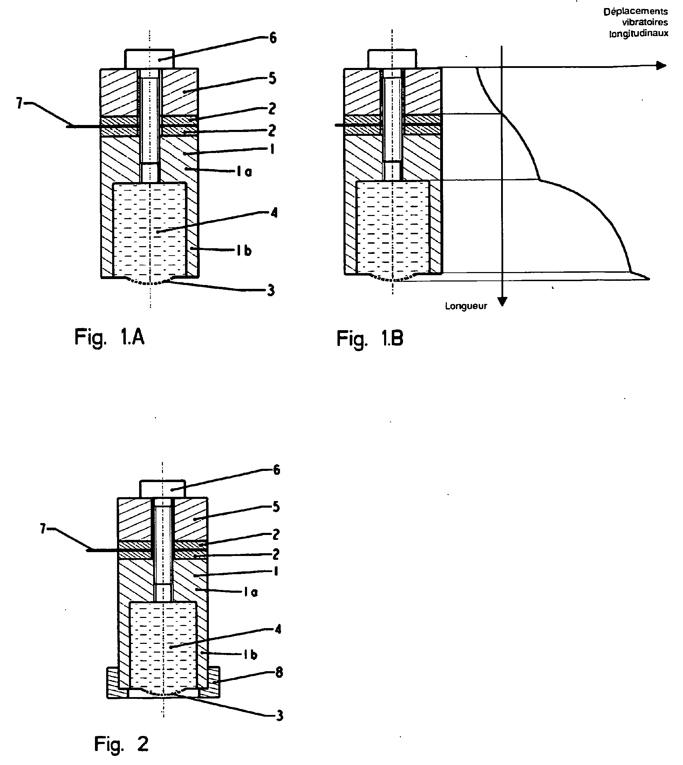

L'atomiseur illustré sur la figure 1A comporte un corps de transducteur piézoélectrique **1** que l'on fait vibrer de préférence dans la gamme 50 kHz à 200 kHz. La figure 1B illustre le même atomiseur mais à côté duquel est illustrée une courbe montrant l'amplitude maximale des déplacements longitudinaux des différentes parties de l'atomiseur. Le corps de transducteur piézoélectrique **1** se caractérise par deux zones : une zone de concentration de contraintes **1a** et une zone d'amplification des déformations **1b.** Dans les figures 1 à 6, le diamètre externe de la zone de concentration de contraintes **1a** est identique au diamètre externe de la zone d'amplification de déformations **1b.** Par contre, le diamètre interne de la zone d'amplification de déformations **1b** est supérieur au diamètre interne de la zone de concentration de contraintes **1a.**

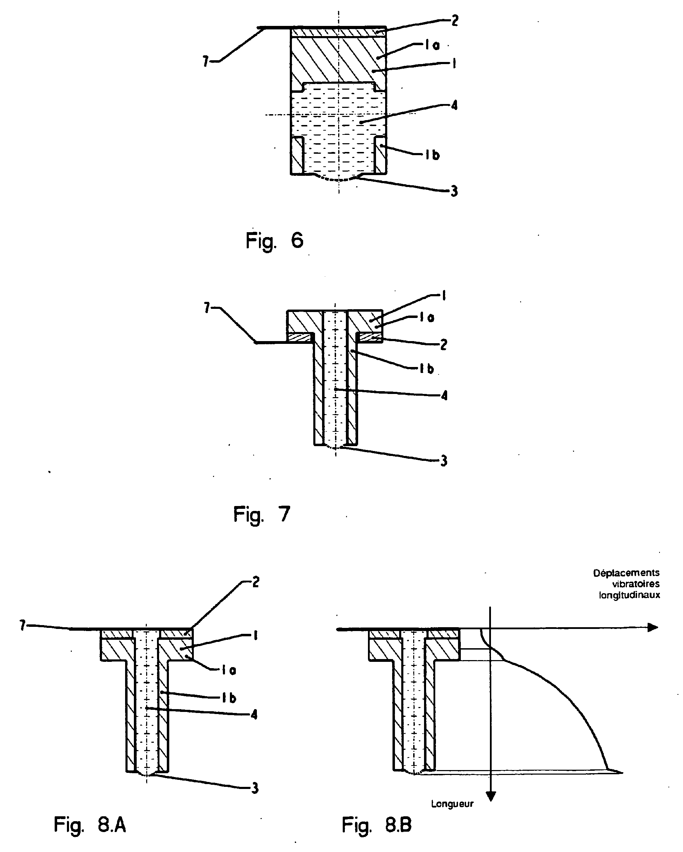

Dans les atomiseurs illustrés sur les figures 7 à 9, le diamètre externe de la zone de concentration de contraintes **1a** est supérieur au diamètre externe de la zone d'amplification de déformations **1b.** Par contre, le diamètre interne de la zone d'amplification de déformations **1b** est identique au diamètre interne de la zone de concentration de contraintes **1a.**

La figure 8B représente le même type d'information que la figure 1B, à savoir une courbe montrant l'amplitude maximale des déplacements longitudinaux des différentes parties de l'atomiseur.

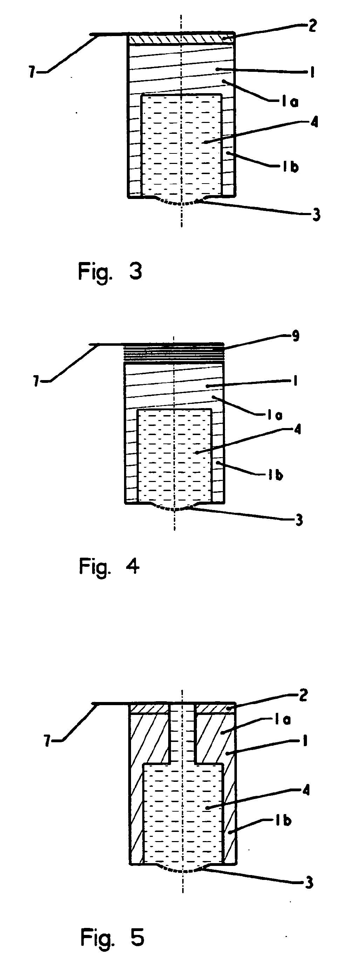

L'intérieur de la zone d'amplification de déformations **1b** est constitué d'une cavité **4** contenant le liquide à atomiseur. Dans certains cas, voir en particulier les figures 5,7 et 8 à 10, la cavité **4** s'étend à l'intérieur de la zone de concentration de contraintes **1a.** Grâce à cette configuration, l'énergie ultrasonore est conservée principalement dans la zone d'amplification de déformations **1b,** ce qui constitue un amplificateur de déplacements vibratoires. La conservation d'énergie dans la zone d'amplification de déformations **1b** implique une conversion des contraintes en déformations.

Un ou plusieurs éléments piézoélectriques, constitués de préférence d'une céramique piézoélectrique monobloc **2** ou multicouche **9,** sont disposés dans la partie supérieure de l'atomiseur, au niveau de la zone de concentration de contraintes **1a.** La figure 1 par exemple représente deux céramiques piézoélectriques monoblocs **2** connectées par une électrode centrale **7,** par exemple en laiton.

La masse arrière **5** (masse dynamique) permet de réduire les déformations à l'arrière des céramiques piézoélectriques. La vis de précontrainte **6** permet de lier mécaniquement l'ensemble de cet empilage. Cet ensemble constitue un transducteur piézoélectrique qui est un convertisseur électromécanique qui vibre suivant un mode longitudinal. Un mode longitudinal est défini par le fait que le transducteur se déforme suivant son axe de symétrie par élongation ou contraction de sa section. Le comportement vibratoire de ce type de transducteur est gouverné essentiellement par sa longueur de telle manière que le ratio longueur total du transducteur sur le diamètre ou largeur de la céramique piézoélectrique est, préférentiellement, supérieur ou égal à 1.

La membrane micro-perforée **3** ou une grille de faible épaisseur (20 à 200 µm) est fixée mécaniquement au bout du corps de transducteur piézoélectrique 1 où sa vitesse vibratoire est maximale. La fixation de la membrane **3** est telle que celle-ci est couplée acoustiquement au transducteur dans 1a zone **1b.** Dans un premier mode de déformation et à titre d'exemple, ce transducteur se déforme et vibre suivant sa demi-longueur d'onde. La figure 1B montre l'évolution du déplacement des points du transducteur dans une section suivant son axe de symétrie (longueur).

La figure 2 montre ce même atomiseur muni d'une membrane liée mécaniquement et acoustiquement au transducteur via un élément de liaison **8** permettant d'assurer une forte précontrainte sur la membrane. De manière générale, la membrane peut être couplée mécaniquement à la zone d'amplification par collage, par brasage, par sertissage ou par soudage. En particulier, le soudage laser peut être utilisé.

La figure 3 est une variante du transducteur ou la masse arrière est éliminée pour des raisons de simplicité de construction. Le transducteur est dimensionné de telle manière que le déplacement au niveau de la céramique piézoélectrique monobloc **2** soit le plus faible possible et le plus grand possible dans la zone d'amplification **1b.** L'électrode **7** peut être constituée en fixant un clinquant de laiton par exemple ou en collant un circuit imprimé flexible sur polyimide.

La figure **4** représente une variante de l'invention en utilisant une céramique piézoélectrique multicouche **9.** Les couches ont une épaisseur par exemple de 20 à 200 µm et l'utilisation de tels multicouches permet, à moindre coût, de réduire la tension d'alimentation électrique aux bornes de la céramique. Cette configuration est très intéressante pour les applications exigeant une alimentation sur pile ou batterie.

La figure 5 présente une variante de l'invention ou la cavité (chambre) contenant le liquide **4** traverse le corps du transducteur **1** de part en part dans sa longueur. Dans ce cas, la céramique piézoélectrique monobloc **2** présente un trou en son centre. Cette configuration permet d'alimenter aisément la cavité en liquide.

La figure 6 montre un autre type d'alimentation en liquide en pratiquant des passages, trous ou rainures pour faire communiquer la cavité remplie de liquide **4** avec l'extérieur. Cette configuration permet de placer le réservoir de liquide autour du transducteur.

Dans la forme de réalisation des figures 7 et 8, la cavité contenant le liquide **4** est tubulaire pour des raisons de simplicité de forme.

Dans la configuration de la figure 7, la céramique **2** n'est plus située à l'arrière du corps du transducteur **1** mais au niveau de l'amplificateur de déplacement à l'avant de la zone de concentration de contrainte **1a.** La céramique piézoélectrique monobloc **2** est ainsi protégée par le corps du transducteur **1.**

Cette configuration offre l'avantage de ne pas avoir la céramique **2** en contact avec le liquide et de ne pas poser de problèmes d'étanchéité avec le réservoir. La variation de section du corps du transducteur **1** permet toujours d'amplifier le déplacement vibratoire au niveau de la membrane **3.**

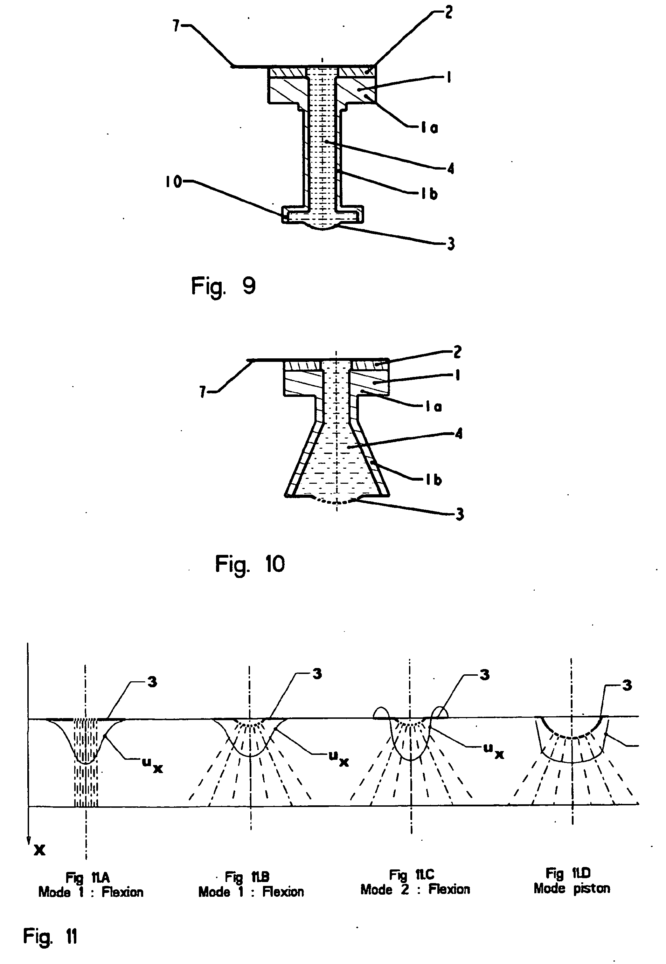

La figure 9 montre une autre forme de réalisation ou le transducteur **1** comporte un pavillon **10** sur lequel est fixée mécaniquement et acoustiquement une membrane micro-perforée **3.** L'avantage de cette configuration est d'augmenter le débit de liquide atomisé par simple effet de surface tout en maintenant un niveau d'amplification de déplacement vibratoire élevé.

La figure 10 illustre, à titre d'exemple, une géométrie en forme de tronc de cône de l'amplificateur de déplacements vibratoire **1b.** Cette configuration permet d'augmenter la dimension de la membrane micro-perforée **3.**

La figure 11 explicite le fonctionnement vibratoire de la membrane micro-perforée **3.** Dans les structures résultantes de l'état antérieur (11A, 11B, 11C), l'atomiseur fonctionne en flexion par couplage d'une céramique piézoélectrique annulaire avec la membrane micro-perforée. Lorsque la membrane est plate (Fig. 11A), le déplacement vibratoire maximal (Uₓ) se situe au centre de la membrane et décroît fortement lorsqu'on s'en éloigne. Dans ce cas, le jet est très directif. Lorsque la membrane est bombée et comporte un dôme (Fig. 11B et 11C), celui-ci rigidifie le mode de vibration et fait diverger par simple effet géométrique le jet. Ceci est observé quel que soit le mode de vibration en flexion considéré. Le mode 1 de flexion étant plus avantageux de ce point de vue. Dans le cas de la présente invention (11D), la géométrie et la nature de la membrane n'influe pas sur le mode de vibration de l'atomiseur. En effet, la raideur en flexion de la membrane n'a aucune influence sur la déformation longitudinale du transducteur. Pour obtenir le meilleur résultat, il suffit que le diamètre du dôme soit très proche de celui du transducteur afin que la membrane suive simplement les déplacements vibratoires maximum à cet endroit. Une telle configuration assure à l'atomiseur un plus grand rendement et donc une moindre consommation pour un débit d'atomisation identique. De plus, le jet est particulièrement homogène et diffus.

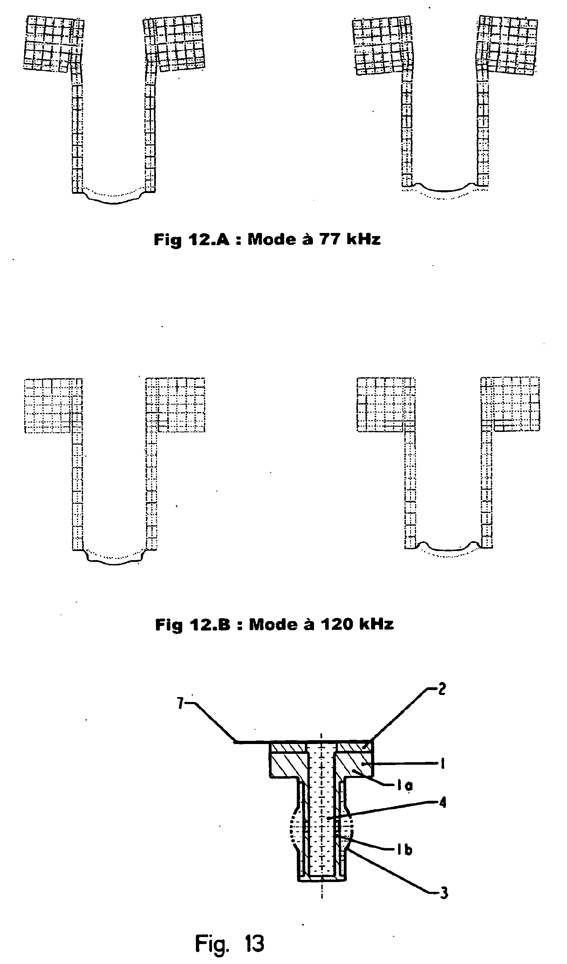

Les figures 12A et 12B montrent les déformées simulées par calculs aux éléments finis d'un atomiseur réalisé selon la présente invention et donné en exemple.

Dans ce cas précis, le corps du transducteur **1** est réalisé en acier inoxydable. La cavité intérieure contenant le liquide **4,** a un diamètre de 6mm et la zone de concentration de contrainte **1a,** a un diamètre extérieur de 16mm.

La zone d'amplification de déformation **1b** a un diamètre extérieur de 8mm. La céramique piézoélectrique monobloc **2** est une céramique PIC 255 (Physic Instruments) de diamètre intérieur 8mm, de diamètre extérieur 16mm et d'épaisseur 1mm.

Les longueurs du transducteur 1 et de la zone d'amplification de déformation **1b** sont respectivement de 16mm et de 12mm. La membrane micro-perforée **3** a été réalisée en Nickel électro-formée munie de 800 trous de 5µm de diamètre. L'épaisseur de la membrane est de 50µm et possède un diamètre extérieur de 8mm. Le dôme a une hauteur de 0,8mm pour un diamètre de 5mm. La membrane est fixée sur le transducteur par collage. Les modes longitudinaux concernés ont respectivement des fréquences de résonance de 77kHz et 120kHz.

Dans la forme de réalisation selon la figure 13, 1a membrane micro-perforée **3** possède une géométrie cylindrique ou tubulaire. La membrane est fixée d'une part sur la zone de concentration de contrainte **1a** et, d'autre part, sur la zone d'amplification de déformation **1b.** Dans ce cas, la membrane vibre suivant un mode radial.

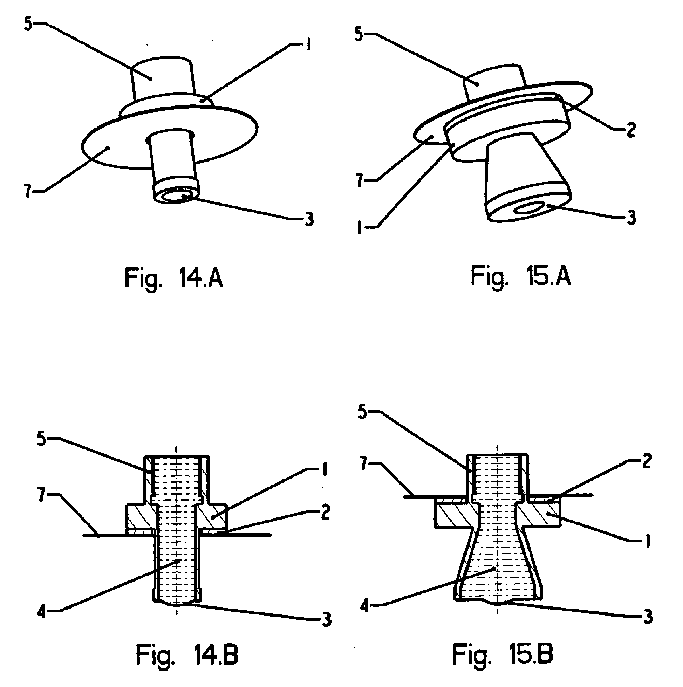

Les figures 14 & 15 montrent des exemples de réalisation qui ont donné d'excellents résultats en termes de taille de gouttelette et de débit d'aérosol. Les figures 14A et 14B décrivent en perspective et en coupe un transducteur 1 dont le corps a été réalisé en acier inoxydable. La cavité intérieure contenant le liquide **4,** a un diamètre de 6 mm et la zone de concentration de contrainte **1a,** un diamètre de 16mm. Le mécanisme de fixation du réservoir se présente, dans cette configuration spécifique, comme une masse arrière **5** dans la quelle, il a été pratiqué un filetage. Le diamètre extérieur et la longueur de cette masse arrière sont respectivement de 10mm et 8mm. La céramique piézoélectrique monobloc **2** est une céramique PIC 255 (Physic Instruments) de diamètre intérieur 8mm, de diamètre extérieur 16mm et d'épaisseur 1mm. La zone d'amplification de déformation **1b** (ou « trompe ») possède un diamètre extérieur de 7mm et une longueur de 12mm.

L'électrode **7** permettant la connexion électrique de la céramique piézoélectrique monobloc **2** est un clinquant en acier inoxydable de 30mm de diamètre et d'épaisseur 50 µm. La membrane **3** en Nickel électro-formée comprend 10800 trous de 2 µm pour une épaisseur de 20 µm. L'atomiseur a permis d'obtenir des gouttelettes de taille 2 µm pour un débit de 0,6 ml/min pour une fréquence de fonctionnement de 80 kHz.

Les figures 15A et 15B décrivent en perspective et en coupe un atomiseur dont le corps du transducteur **1** a été réalisé en acier inoxydable. La cavité intérieure contenant le liquide **4,** a un diamètre qui varie de 6mm à 12mm et la zone de concentration de contrainte **1a** un diamètre de 20mm. Le mécanisme de fixation du réservoir se présente, dans cette configuration spécifique, comme une masse arrière 5 dans la quelle, il a été pratiqué un filetage. Le diamètre extérieur et la longueur de cette masse arrière sont respectivement de 10mm et 8mm. La céramique piézoélectrique monobloc **2** est une céramique PIC 255 (Physic Instruments) de diamètre intérieur 10mm, de diamètre extérieur 20mm et d'épaisseur 1mm. La zone d'amplification de déformation **1b** (ou « trompe ») de forme conique possède un diamètre extérieur qui varie de 7mm à 14mm et une longueur de 9mm.

L'électrode 7 permettant la connexion électrique de la céramique piézoélectrique monobloc **2** est en acier inoxydable de 30mm de diamètre et d'épaisseur 50 µm. La membrane **3** en Nickel électro-formée comprend 45 300 trous de 2 µm pour une épaisseur de 20 µm. L'atomiseur a permis d'obtenir des gouttelettes de taille 2 µm pour un débit de 2,5 ml/min pour une fréquence de fonctionnement de 70 kHz.

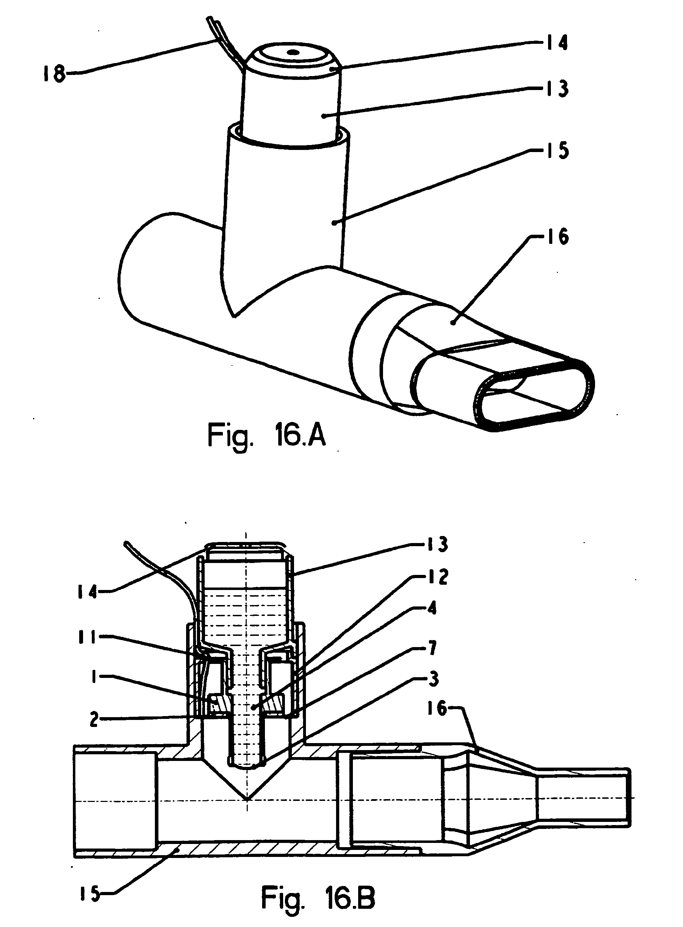

Les figures 16A et 16B décrivent un inhalateur destiné à délivrer des médicaments par voie pulmonaire. Cet inhalateur peut se mettre sous la forme d'un embout buccal **16** associé à un boîtier **15** en forme de T fourni, par exemple, par Intersurgical dans lequel s'intègre l'atomiseur, objet de la présente invention. L'atomiseur est placé dans le boîtier **15** à l'aide d'un cache transducteur **12.** Le réservoir **13** muni de son bouchon **14** porte le transducteur **1.** La céramique piézoélectrique monobloc **2** est alimentée par l'électrode **7** sous forme d'un clinquant. L'atomiseur est alimenté par des câbles **18.** Lorsque l'atomiseur fonctionne, celui-ci génère un aérosol à l'intérieur du boîtier **15.** Le patient inhale l'aérosol ainsi généré, via l'embout buccal **16.**

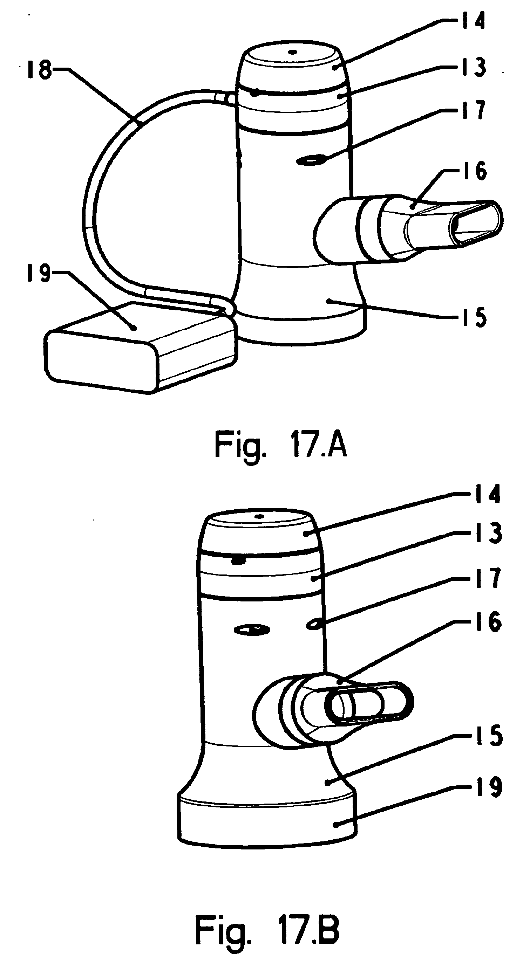

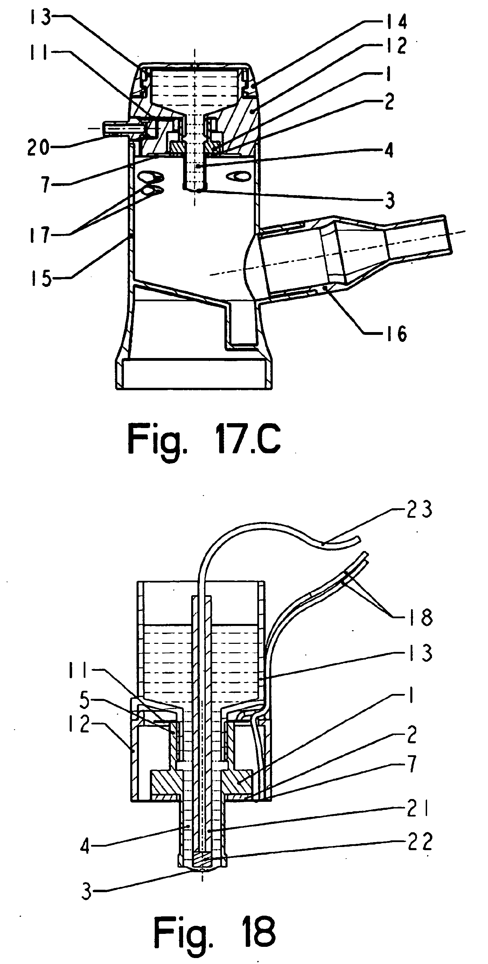

Les figures 17 décrivent une autre forme d'inhalateur. La figure 17A représente un inhalateur intégrant l'atomiseur objet de la présente invention ou le boîtier électronique **19** est placé à l'extérieur et est relié au boîtier **15** de l'inhalateur par un câble **18.** Cet inhalateur comprend un embout buccal **16,** un réservoir **13** lié à l'atomiseur et un bouchon **14.** Des ouvertures **17** ont été pratiquées dans le boîtier **15** pour gérer les flux d'air et l'aérosol produit par l'atomiseur.

La figure 17B représente un inhalateur ou le boîtier électronique **19** est intégré dans le boîtier **15** de l'inhalateur de « poche ».

La figure 17C représente, en coupe, l'inhalateur de la figure 17A. Nous retrouvons le boîtier **15** fabriqué par exemple en plastique moulé, l'embout **16** qui peut être amovible et jetable après utilisation, le cache transducteur **12** qui permet de monter l'atomiseur dans le boîtier **15** et le connecter au boîtier électronique **19** via le connecteur **20** et le câble **18.** L'atomiseur est constitué du corps du transducteur **1,** de la céramique piézoélectrique monobloc **2,** de la membrane vibrante **3,** de l'électrode **7,** du retour de masse **11,** du réservoir **13** et du bouchon **14.** L'aérosol est généré dans la cavité du boîtier **15** et aspiré par le patient à travers l'embout buccal **16.**

La figure 18 est une coupe d'un atomiseur muni d'un tube permettant l'évacuation des bulles d'air et d'un capteur de présence de liquide. Cet atomiseur comprend un corps de transducteur tubulaire **1,** une céramique piézoélectrique monobloc **2,** une membrane vibrante micro-perforée **3** et une masse arrière **5** permettant de fixer le réservoir **13.** La céramique piézoélectrique monobloc **2** est alimentée par un câble **18** connecté d'une part au retour de masse **11** et à l'électrode **7.** Un tube **21,** préférentiellement en plastique et par exemple de diamètre 3mm, est placé dans la cavité de liquide **4** de manière coaxiale. Lorsque le débit liquide de l'atomiseur devient important, la membrane **3** crée une dépression telle que de l'air peut pénétrer à l'intérieur de la cavité contenant le liquide **4.** La formation de bulles d'air au niveau de la membrane **3** peut bloquer la formation de l'aérosol et altère le fonctionnement de l'atomiseur. Le tube **21** permet l'évacuation des bulles d'air par l'action des forces capillaires qu'il exerce sur l'interface air-liquide. Ce même tube **21** comprend en son centre un fil conducteur électrique **23** dont l'extrémité **22** est en contact électriquement directement ou indirectement avec le liquide. Un signal électrique alternatif basse fréquence préférentiellement à 500 Hz est transmis au fil conducteur **23.** La membrane **3** ainsi que le corps du transducteur **1,** n'étant pas au même potentiel électrique, il en résulte un courant dû à la résistivité du liquide présent dans la cavité **4.** L'existence de ce courant correspond à la présence du liquide. L'information provenant du capteur de présence de liquide **22** permet de démarrer ou d'arrêter le fonctionnement de l'atomiseur automatiquement.

L'invention ne se limite bien évidemment pas aux exemples discutés ci-dessus. De même, elle ne se limite pas qu'au domaine médical. L'atomiseur selon l'invention peut également être utilisé comme diffuseur d'odeurs et de parfum et/ou dans l'application de produits cosmétiques. L'invention couvre également la diffusion de brouillards de liquides variés à usage local (humidificateurs ou lubrificateurs) ou les dispositifs de manipulation de liquides pour les biotechnologies ou les réactifs.

## Revendications

1. Atomiseur de liquide par ultrasons comprenant :
- un corps de transducteur piézoélectrique (1) rigide comprenant une première extrémité formant une ouverture et une deuxième extrémité, l'intérieur du corps de transducteur piézoélectriques (1) comprenant une cavité destinée à contenir un liquide à atomiser, ledit corps (1) comportant en outre un axe de symétrie,
- une membrane micro-perforée (3) fixée sur ladite première extrémité et recouvrant ladite ouverture,
- un élément piézoélectriques (2,9) adapté et disposé de manière à faire vibrer le corps de transducteur piézoélectrique (1),
**caractérisé par le fait que** l'élément piézoélectrique (2,9) est disposé vers ladite deuxième extrémité, de manière à faire vibrer le corps de transducteur piézoélectriques (1) selon une direction parallèle à son axe de symétrie.

2. Atomiseur selon la revendication 1 dans lequel la section du corps de transducteur piézoélectrique (1) varie sur sa longueur.

3. Atomiseur selon la revendication 2 dans lequel la section varie de manière discontinue.

4. Atomiseur selon la revendication 3 dans lequel la section varie brusquement en un seul endroit.

5. Atomiseur selon la revendication 4 dans lequel l'épaisseur des parois du corps de transducteur piézoélectrique (1) vers ladite deuxième extrémité est supérieure à l'épaisseur des parois du corps de transducteur piézoélectrique (1) vers la première extrémité.

6. Atomiseur selon la revendication 5 dans lequel le corps de transducteur piézoélectrique est plein vers ladite deuxième extrémité.

7. Atomiseur selon la revendication 5 dans lequel le corps de transducteur piézoélectrique est creux vers ladite deuxième extrémité.

8. Atomiseur selon l'une des revendications 2, 3 ou 4 dans lequel le diamètre interne le corps de transducteur piézoélectrique (1) est constant.

9. Atomiseur selon l'une des revendications 2, 3 ou 4 dans lequel le diamètre interne du corps de transducteur piézoélectrique (1) est variable.

10. Atomiseur selon la revendication 9 dans lequel le diamètre interne vers ladite première extrémité est supérieur au diamètre interne vers ladite deuxième extrémité.

11. Atomiseur selon l'une quelconque des revendications précédentes dans lequel l'élément piézoélectrique (2,9) est disposé à l'arrière du corps du transducteur (1) ou à l'avant de la zone de concentration de contrainte.

12. Atomiseur selon la revendication 11 comprenant une masse arrière (5) disposée contre la face externe de l'élément piézoélectrique (2,9).

13. Atomiseur selon l'une quelconque des revendications 1 à 10 dans lequel le diamètre externe du corps de transducteur piézoélectrique (1) est constant.

14. Atomiseur selon l'une quelconque des revendications 1 à 10 dans lequel le diamètre externe du corps de transducteur piézoélectrique (1) est variable.

15. Atomiseur selon la revendication 14 dans lequel le diamètre externe vers ladite deuxième extrémité est supérieur au diamètre externe vers ladite première extrémité.

16. Atomiseur selon la revendication 15 dont la face externe du corps de transducteur piézoélectrique est définie par un premier diamètre et un deuxième diamètre, la zone de transition entre les deux diamètres formant un décrochement brusque.

17. Atomiseur selon la revendication 16 dans lequel l'élément piézoélectrique (2) est disposé dans le décrochement et s'appuie sur la portion du corps de transducteur piézoélectrique (1) qui comporte la deuxième extrémité.

18. Atomiseur selon l'une quelconque des revendications précédentes dans lequel le rapport entre la longueur et le diamètre de ladite cavité est supérieur à 0.5.

19. Atomiseur selon l'une quelconque des revendications précédentes dans lequel la membrane micro-perforée (3) forme au moins partiellement une protubérance permettant de la rigidifier.

20. Atomiseur selon l'une quelconque des revendications précédentes dans lequel la membrane micro-perforée (3) à une épaisseur entre 20 et 200 µm et comporte des trous de diamètre entre 1 µm et 100 µm.

21. Atomiseur selon l'une quelconque des revendications précédentes dans lequel l'élément piézoélectrique est une céramique multicouche (9).

## Claims

1. Ultrasound liquid atomizer comprising:
- a rigid piezoelectric transducer body (1) having a first end defining an opening and a second end, the inside of the piezoelectric transducer body (1) comprising a cavity for containing a liquid to be atomized and said body (1) further comprising a symmetry axis,
- a micro-perforated membrane (3) attached to said first end and covering said opening,
- a piezoelectric member (2, 9) adapted and arranged so as to vibrate the piezoelectric transducer body (1),
**characterized in that** the piezoelectric member (2, 9) is located toward said second end in order to vibrate the piezoelectric transducer body (1) in a direction parallel to its symmetry axis.

2. Atomizer according to Claim 1, wherein the section of the piezoelectric transducer body (1) varies over its length.

3. Atomizer according to Claim 2, wherein the section varies discontinuously.

4. Atomizer according to Claim 3, wherein the section varies abruptly at a single point.

5. Atomizer according to Claim 4, wherein the thickness of the walls of the piezoelectric transducer body (1) toward said second end is greater than the thickness of the walls of the piezoelectric transducer body (1) toward the first end.

6. Atomizer according to Claim 5, wherein the piezoelectric transducer body is solid toward said second end.

7. Atomizer according to Claim 5, wherein the piezoelectric transducer body is hollow toward said second end.

8. Atomizer according to one of Claims 2, 3 or 4, wherein the internal diameter of the piezoelectric transducer body (1) is constant.

9. Atomizer according to one of Claims 2, 3 or 4, wherein the internal diameter of the piezoelectric transducer body (1) is variable.

10. Atomizer according to Claim 9, wherein the internal diameter toward said first end is greater than the internal diameter toward said second end.

11. Atomizer according to any one of the preceding claims, wherein the piezoelectric member (2, 9) is located at the rear of the body of the transducer 1 or in front of the stress concentration zone.

12. Atomizer according to Claim 11, comprising a rear mass (5) located against the external face of the piezoelectric member (2, 9).

13. Atomizer according to any one of Claims 1 to 10, wherein the external diameter of the piezoelectric transducer body (1) is constant.

14. Atomizer according to any one of Claims 1 to 10, wherein the external diameter of the piezoelectric transducer body (1) is variable.

15. Atomizer according to Claim 14, wherein the external diameter toward said second end is greater than the external diameter toward said first end.

16. Atomizer according to Claim 15, of which the external face of the piezoelectric transducer body is defined by a first diameter and a second diameter, the transition zone between the two diameters forming an abrupt discontinuity.

17. Atomizer according to Claim 16, wherein the piezoelectric member (2) is positioned in the discontinuity and bears on the portion of the piezoelectric transducer body (1) that includes the second end.

18. Atomizer according to any one of the preceding claims, wherein the ratio between the length and the diameter of said cavity is greater than 0.5.

19. Atomizer according to any one of the preceding claims, wherein the micro-perforated membrane (3) at least partially forms a protuberance making it possible to increase its rigidity.

20. Atomizer according to any one of the preceding claims, wherein the micro-perforated membrane (3) has a thickness between 20 and 200 µm and includes holes of a diameter between 1 µm and 100 µm.

21. Atomizer according to any one of the preceding claims, wherein the piezoelectric member is a multi-layer ceramic (9).

## Patentansprüche

1. Ultraschall-Flüssigkeitszerstäuber, der Folgendes aufweist:
- einen starren Piezowandlerkörper (1), der ein erstes Ende, das eine Öffnung bildet, und ein zweites Ende aufweist, wobei das Innere des Piezowandlerkörpers (1) einen Hohlraum aufweist, der dazu bestimmt ist, eine zu zerstäubende Flüssigkeit zu enthalten, wobei der Körper (1) ferner eine Symmetrieachse aufweist,
- eine mikroperforierte Membran (3), die auf dem ersten Ende befestigt ist und die Öffnung bedeckt,
- ein Piezoelement (2, 9), das geeignet und derart angeordnet ist, dass es den Piezowandlerkörper (1) zum Schwingen bringt,
**dadurch gekennzeichnet, dass** das Piezoelement (2, 9) derart in Richtung des zweiten Endes angeordnet ist, dass es den Piezowandlerkörper (1) in einer Richtung zum Schwingen bringt, die parallel zu seiner Symmetrieachse ist.

2. Zerstäuber nach Anspruch 1, wobei der Querschnitt des Piezowandlerkörpers (1) über seine Länge variiert.

3. Zerstäuber nach Anspruch 2, wobei der Querschnitt auf diskontinuierliche Weise variiert.

4. Zerstäuber nach Anspruch 3, wobei der Querschnitt an einer einzigen Stelle abrupt variiert.

5. Zerstäuber nach Anspruch 4, wobei die Dicke der Wände des Piezowandlerkörpers (1) in Richtung des zweiten Endes größer ist als die Dicke der Wände des Piezowandlerkörpers (1) in Richtung des ersten Endes.

6. Zerstäuber nach Anspruch 5, wobei der Piezowandlerkörper in Richtung des zweiten Endes vollständig ausgefüllt ist.

7. Zerstäuber nach Anspruch 5, wobei der Piezowandlerkörper in Richtung des zweiten Endes hohl ist.

8. Zerstäuber nach einem der Ansprüche 2, 3 oder 4, wobei der Innendurchmesser des Piezowandlerkörpers (1) gleichbleibend ist.

9. Zerstäuber nach einem der Ansprüche 2, 3 oder 4, wobei der Innendurchmesser des Piezowandlerkörpers (1) veränderlich ist.

10. Zerstäuber nach Anspruch 9, wobei der Innendurchmesser in Richtung des ersten Endes größer ist als der Innendurchmesser in Richtung des zweiten Endes.

11. Zerstäuber nach einem der vorhergehenden Ansprüche, wobei das Piezoelement (2, 9) hinter dem Körper (1) des Wandlers oder vor dem Spannungskonzentrationsbereich angeordnet ist.

12. Zerstäuber nach Anspruch 11, der eine hintere Masse (5) aufweist, die gegen die Außenseite des Piezoelements (2, 9) angeordnet ist.

13. Zerstäuber nach einem der Ansprüche 1 bis 10, wobei der Außendurchmesser des Piezowandlerkörpers (1) gleichbleibend ist.

14. Zerstäuber nach einem der Ansprüche 1 bis 10, wobei der Außendurchmesser des Piezowandlerkörpers (1) veränderlich ist.

15. Zerstäuber nach Anspruch 14, wobei der Außendurchmesser in Richtung des zweiten Endes größer ist als der Außendurchmesser in Richtung des ersten Endes.

16. Zerstäuber nach Anspruch 15, wobei die Außenseite des Piezowandlerkörpers durch einen ersten Durchmesser und einen zweiten Durchmesser abgegrenzt ist, wobei der Übergangsbereich zwischen den zwei Durchmessern einen abrupten Absatz bildet.

17. Zerstäuber nach Anspruch 16, wobei das Piezoelement (2) im Absatz angeordnet ist und auf dem Abschnitt des Piezowandlerkörpers (1) aufliegt, der das zweite Ende aufweist.

18. Zerstäuber nach einem der vorhergehenden Ansprüche, wobei das Verhältnis zwischen der Länge und dem Durchmesser des Hohlraums größer als 0,5 ist.

19. Zerstäuber nach einem der vorhergehenden Ansprüche, wobei die mikroperforierte Membran (3) mindestens teilweise einen Vorsprung bildet, der ihr Versteifen ermöglicht.

20. Zerstäuber nach einem der vorhergehenden Ansprüche, wobei die mikroperforierte Membran (3) eine Dicke zwischen 20 und 200 µm aufweist und Löcher mit einem Durchmesser zwischen 1 µm und 100 µm aufweist.

21. Zerstäuber nach einem der vorhergehenden Ansprüche, wobei das Piezoelement eine Mehrschicht-Keramik (9) ist.
